# EUROPEAN PATENT APPLICATION

(11) **EP 1 726 275 A2**
(43) Date of publication of application: **29.11.2006**
(21) Application number: 06018552.7
(22) Date of filing: 28.04.2004
(51) Int. Cl.: A61F 5/44

(54) **Medical waste collection device**

(30) Priority: 29.04.2003 GB 0309800
(62) Divisional of application: 04252458.7
(71) Applicant: Mentor Medical Limited, Lancing, West Sussex BN15 8TJ (GB)
(72) Inventor: French, Nigel, Lancing West Sussex BN15 8TJ (GB)
(74) Representative: Driver, Virginia Rozanne

(57) **Abstract**

A medical waste collection device, including a collection bag having an inlet and a separate outlet, and a closed tap at the outlet of the bag to block, in use, the flow of waste fluid through the outlet, the dosed tap being controllably openable to allow, in use, fluid collected in the bag to be controllably drained from the bag, but being less easily reclosable to thereby deter reuse of the tap and bag.

## Description

The present invention relates to a waste collection device, particularly to a waste collection bag device for collecting fluids, such as urine, discharged from a human body.

Waste collection bags of the kind used in the medical field can by virtue of their construction be drained by either tearing or cutting the bag thereby at the same time preventing reuse of the bag.

It is an aim of the present invention to provide an improved construction for a waste collection bag device that is intended to be disposed of after a single use.

The present invention provides a medical waste collection device, including a collection bag having an inlet and a separate outlet, and a closed tap at the outlet of the bag to block, in use, the flow of waste fluid through the outlet, the closed tap being controllably openable to allow, in use, fluid collected in the bag to be controllably drained from the bag, but being less easily reclosable to thereby deter reuse of the tap and bag.

The present invention also provides the use of such a waste collection device for collecting fluids discharged from a human body.

An embodiment of the present invention is described hereunder, by way of example only, with reference to the accompanying drawings, in which:-
Figure 1 shows a waste collection device according to an embodiment of the present invention with the tap in a closed position; and
Figure 2 shows the waste collection device of Figure 1 with the tap in the open position.

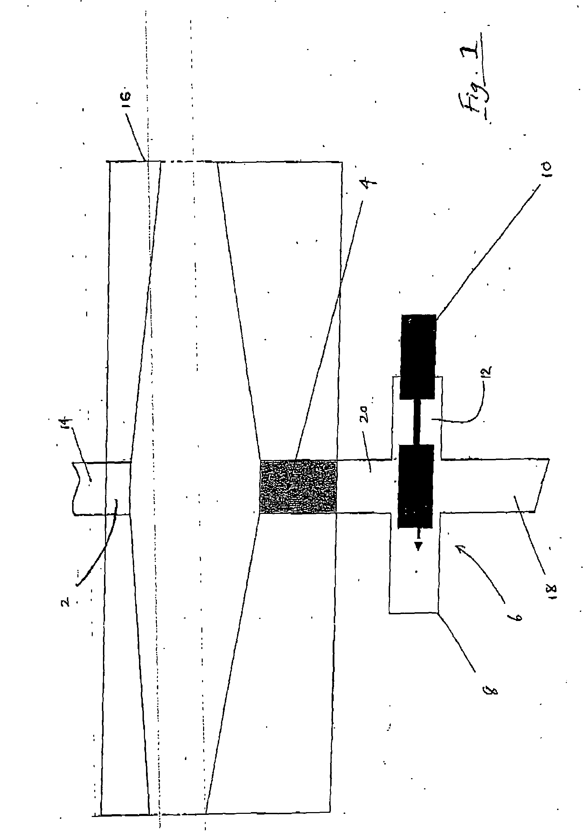

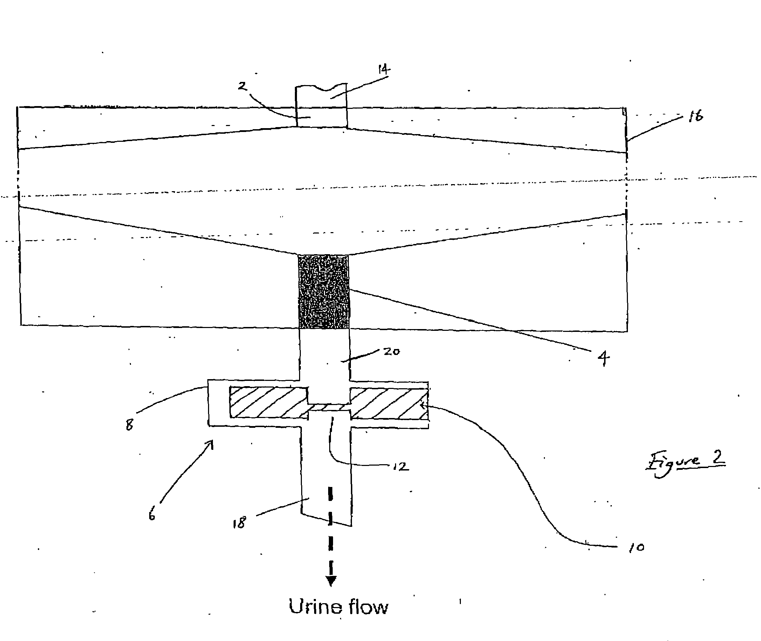

With reference to Figures 1 and 2, a waste collection device according to an embodiment of the present invention includes a collection bag 16 having an inlet at the top end and an outlet 4 at the bottom end. Into the inlet 2 is permanently welded inlet hosing or tubing 14 for leading the waste fluid from the human body into the collection bag. Into the outlet 4 is permanently welded a tap 6. The tap includes a housing 8 which defines an outlet 18 and an inlet 20 which is connected to the interior of the collection bag via the bag outlet 4. In the housing is fitted a sliding actuator 10. The sliding actuator 10 includes a section of reduced diameter 12 which allows the flow of water from the inlet to the outlet when (at least partially) aligned with the inlet and outlet of the housing. The sliding actuator 10 is fitted in the housing 8 such that the actuator blocks the flow of fluid from the inlet to the outlet other than when the section of reduced diameter 12 is (at least partially) aligned with the inlet and outlet of the housing.

In use, the waste collection device is supplied with the tap in a closed position as shown in Figure 1. The inlet hosing or tubing is connected to the patient for directing waste fluid into the collection bag. The close fit of the actuator in the housing such that there is sufficient frictional force between the two components to prevent the tap being accidentally dislodged into the open position.

When the collection bag is full or all the fluid to be collected has been collected, the tap is opened by pushing the actuator in the direction shown by the arrow in Figure 1 via the portion of the actuator protruding from the housing 8. As shown in Figure 2, the tap is designed such that when the actuator has been pushed into the housing as far as it can be easily done so by hand, i.e. when the end of the actuator 10 is substantially aligned with the opening of the housing 8, the whole of the section of the actuator of reduced diameter 12 is substantially aligned with the outlet and inlet of the housing.

The housing 8 is closed at the other end such that the actuator 10 cannot be pushed in the opposite direction back into a closed position. The closed end of the housing is positioned such that it abuts with the respective end of the actuator when the tap is in the "open" position, thereby serving to also further prevent any further undesirable insertion of the actuator to a position where the flow of fluid is more restricted or completely blocked.

With this tap construction, it is very difficult for a user to inadvertently reuse the tap/bag/inlet tubing assembly, which reuse would generally be undesirable from the point of view of avoiding accidental contamination. With this waste collection device, the bag can be controllably drained by hand whilst at the same time effectively deterring reuse of the tap/bag/inlet tubing assembly.

Furthermore, the tap is of a simple construction which can be produced and incorporated into a collection bag at minimal cost whilst providing the above-mentioned advantages.

In one alternative embodiment, the tap is secured into the bag by mechanical means rather than welding. The tap could be a push fit into a tube previously welded into the bag.

In another alternative embodiment, the longitudinal actuator could be a solid rod provided with a transverse throughhole instead of a section of reduced diameter.

The applicant draws attention to the fact that the present invention may include any feature or combination of features disclosed herein either implicitly or explicitly or any generalisation thereof, without limitation to the scope of any definitions set out above. In view of the foregoing description it will be evident to a person skilled in the art that various modifications may be made within the scope of the invention.

## Claims

1. A medical waste collection device, including a collection bag having an inlet and a separate outlet, and a closed tap at the outlet of the bag to block, in use, the flow of waste fluid through the outlet, the closed tap being controllably openable to allow, in use, fluid collected in the bag to be controllably drained from the bag, but being less easily reclosable to thereby deter reuse of the tap and bag.

2. A medical waste collection device according to claim 1, wherein the tap is not reclosable by hand to thereby further deter reuse of the tap and bag.

3. A medical waste collection device according to claim 1, wherein the closed tap includes a housing and a longitudinal insert fitted in said housing in a first position in which the flow of fluid through the tap is blocked and a first longitudinal end of the insert protrudes from the housing; wherein the insert can be pushed longitudinally via the protruding first end thereof to a second position in which fluid can flow through the tap, and wherein the housing is structured so as to prevent access by hand to the other longitudinal end of the insert and thereby deter push operation of the insert in the opposite direction back to a closed position.

4. A medical waste collection device according to claim 3, wherein the portion of the housing receiving said other longitudinal end of the insert is closed so as to prevent access by hand to said other longitudinal end of the insert.

5. A medical waste collection device according to claim 3 or claim 4, wherein when the insert is fully pushed into the housing, the tap is in an open position.

6. The use of a waste collection device according to any preceding claim for collecting fluids discharged from a human body.
